# EUROPÄISCHE PATENTSCHRIFT

(11) **EP 0 987 006 B1**
(45) Veröffentlichungstag und Bekanntmachung des Hinweises auf die Patenterteilung: **03.01.2007**
(21) Anmeldenummer: 99116874.1
(22) Anmeldetag: 06.09.1999
(51) Int. Cl.: A61Q 1/02, A61Q 1/14, A61Q 15/00, A61Q 17/04, A61Q 19/00, A61K 8/06, A61K 8/27, A61K 8/29, A61K 8/35, A61K 8/49, A61K 8/73, A61K 8/81

(54) **Emulgatorfreie feindisperse Systeme vom Typ Wasser-in-Öl**
Emulsifier-free finely dispersed systems of the water-in-oil type
Systèmes en dispersion fine, sans émulsifiants, de type eau-dans-huile

(30) Priorität: 18.09.1998 DE 19842788
(43) Veröffentlichungstag der Anmeldung: 22.03.2000
(73) Patentinhaber: Beiersdorf Aktiengesellschaft, 20245 Hamburg (DE)
(72) Erfinder: Gers-Barlag, Heinrich, Dr., 25495 Kummerfeld (DE); Müller, Anja, 23843 Rümpel (DE)

(56) Entgegenhaltungen:
- EP-A- 0 392 426
- EP-A- 0 529 396
- DE-A- 4 425 268
- US-A- 5 788 952

## Beschreibung

Die vorliegende Erfindung betrifft emulgatorfreie feindisperse Pickering Systeme vom Typ Wasser-in-Öl, bevorzugt als kosmetische oder dermatologische Zubereitungen.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Ver bindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

Kosmetische Zubereitungen werden im wesentlichen zur Hautpflege benutzt. Hautpflege im kosmetischen Sinn ist in erster Linie, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Kosmetische Zubereitungen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Medizinische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, daß bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel. Aus der US 5 788 952 sind kosmetische Sonnenschutzformulierungen in Form von Hydrodisposionen bekannt, die anorganische oberflächlich hydrophobisierte Mikropigmente enthalten.

Pickering stellte um 1910 erstmals Paraffin-Wasser-Emulsionen her, die nur durch den Zusatz verschiedener Feststoffe wie basisches Kupfersulfat, basisches Eisensulfat oder andere Metallsulfate stabilisiert wurden. Diese Art von Emulsionen wird daher auch als Pickering-Emulsion bezeichnet.

Sozusagen die Urformen von Pickering-Emulsionen tauchten zunächst als unerwünschte Nebeneffekte bei unterschiedlichen technischen Prozessen auf, wie z. B. bei der sekundären Erdölförderung, der Extraktion von Bitumen aus Teersand und anderen Trennungsverfahren, an denen zwei nicht miteinander mischbare Flüssigkeiten und feine, dispergierte Feststoffpartikel beteiligt sind. Im allgemeinen handelt es sich hierbei um W/O-Emulsionen, die durch mineralische Feststoffe stabilisiert werden. Dementsprechend stand die Untersuchung entsprechender Systeme, wie z. B. der Systeme Öl-Wasser-Ruß oder Öl-Wasser-Schieferstaub zunächst im Mittelpunkt der Forschungsaktivitäten.

Grundlegende Untersuchungen haben dabei gezeigt, daß ein Charakteristikum für eine Pickering-Emulsion ist, daß die Feststoffpartikel an der Grenzfläche zwischen den beiden flüssigen Phasen angeordnet sind und dort sozusagen eine mechanische Barriere gegen die Vereinigung der Flüssigkeitströpfen bilden.

Eine relativ neue technische Entwicklung ist es, Pickering-Emulsionen als Grundlage für kosmetische oder dermatologische Zubereitungen zu verwenden.

Eine Möglichkeit, eine Feststoffstabilisierung im Sinne einer Pickering-Emulsion in einer kosmetischen oder dermatologischen Zubereitung vorzunehmen, ist nach May-Alert (*Pharmazie in unserer Zeit, 15. Jahrg. 1986, Nr. 1, 1-7*) beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung erreichen.

Die Europäische Offenlegungsschrift 0 686 391 beschreibt darüberhinaus "Emulsionen" vom Typ Wasser-in-Öl, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 µm haben, welche in die Ölpase gegeben werden und diese verdicken. Diese Art von Zubereitungen kann man daher als Öl-Gele (auch: Oleogele) bezeichnen, in welche Wasser stabil eindispergiert werden kann.

Pickering-Emulsionen werden durch den Einsatz von geeigneten Feststoffen bzw. Pigmenten stabilisiert. Allerdings haben die Zubereitungen des Standes der Technik im allgemeinen den Nachteil, daß sie auf einen engen Anwendungsbereich oder eine eingeschränkte Rohstoffauswahl begrenzt sind, da sie sich nur auf diese Weise stabil formulieren lassen. Für einige Bereiche der Kosmetik (z. B. für den Bereich der Gesichtspflege) weisen Pickering-Emulsionen des Standes der Technik unbefriedigende kosmetische Eigenschaften auf.

Aufgabe war daher, den Nachteilen des Standes der Technik Abhilfe zu schaffen. Insbesondere sollten kosmetische und dermatologische Grundlagen für kosmetische und dermatologische Zubereitungen zur Verfügung gestellt werden, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen oder Deodorantien, aber auch ausgesprochen medizinisch-pharmazeutische Darreichungsformen geschaffen werden, zum Beispiel Zubereitungen gegen Akne und andere Hauterscheinungen.

Es war erstaunlich und für den Fachmann in keiner Weise vorauszusehen, daß Pickering-Emulsionen, welche feindisperse Systeme vom Typ Wasser-in-Öl darstellen, enthaltend
(1) eine Ölphase,
(2) eine Wasserphase,
(3) mindestens einen Typ mikrofeiner Partikel, die
   a) eine mittlere Partikelgröße von weniger als 200 nm haben, die
   b) sowohl hydrophile als auch lipophile Eigenschaften zeigen, welche also amphiphilen Charakter besitzen und sowohl in Wasser als auch in Öl dispergierbar sind und die
   c) gegebenenfalls oberflächlich beschichtet sind,
(4) mindestens ein Hydrokolloid und
(5) höchstens 0,5 Gew.-% eines oder mehrerer Emulgatoren
den Nachteilen des Standes der Technik abhelfen.

Es ist erfindungsgemäß besonders vorteilhaft, wenn die Zubereitungen deutlich weniger als 0,5 Gew.-% eines oder mehrerer Emulgatoren enthalten. Ganz besonders bevorzugt sind erfindungsgemäße Zubereitungen, welche gänzlich frei von Emulgatoren im herkömmlichen Sinn sind.

Die erfindungsgemäßen Zubereitungen sind in jeglicher Hinsicht überaus befriedigende Präparate, die gegenüber herkömmlichen Pickering-Emulsionen eine deutlich höhere Stabilität haben und daher insbesondere geeignet sind, um als Grundlage für Zubereitungsformen mit vielfältigen Anwendungszwecken zu dienen. W/O-Pickering-Emulsionen im Sinne der vorliegenden Erfindung sind erstaunlicherweise ausgesprochen stabil.

Zudem zeichnen sich die erfindungsgemäßen Zubereitungen durch eine ausgezeichnete Hautverträglichkeit aus. Ferner war erstaunlich, daß erfindungsgemäße Zubereitungen, die in der Form eines Sonnenschutzmittels vorliegen, eine höhere Wirksamkeit zeigen als übliche Sonnenschutzformulierungen.

Während Zubereitungen des Standes der Technik bereits mit einem Pigmentgehalt von 1 Gew.-% ein stumpfes Gefühl nach ihrer Anwendung auf der Haut erzeugen, das mit höheren Pigmentkonzentrationen noch zunimmt, hinterlassen die erfindungsgemäßen Zubereitungen überraschend keinen trockenen oder stumpfen Eindruck auf der Haut, sondern zeigen vielmehr hervorragende kosmetische Eigenschaften.

Zwar kennt der Stand der Technik neben Pickering-Emulsionen emulgatorfreie, feindisperse kosmetische oder dermatologische Zubereitungen, die im allgemeinen als Hydrodispersionen bezeichnet werden und welche Dispersionen einer flüssigen, halbfesten oder festen inneren (diskontinuierlichen) Lipidphase in einer äußeren wäßrigen (kontinuierlichen) Phase darstellen. Allerdings stellen diese Zubereitungen Öl-in-Wasser-Formulierungen dar.

Der Stand der Technik konnte daher nicht den Weg zur vorliegenden Erfindung weisen.

W/O-Pickering-Emulsionen im Sinne der vorliegenden Erfindung sind durch Dispergieren von erfindungsgemäßen, für die Herstellung von W/O-Pickering-Emulsionen geeigneten amphiphilen Partikeln in der Fettphase erhältlich.

"Hydrokolloid" ist die technologische Kurzbezeichnung für die an sich richtigere Bezeichnung hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken.

Die Gruppe der Hydrokolloide läßt sich wie folgt einteilen in:
- organische, natürliche Verbindungen, wie beispielsweise Agar-Agar, Carrageen, Tragant, Gummi arabicum, Alginate, Pektine, Polyosen, Guar-Mehl, Johannisbrotbaumkemmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 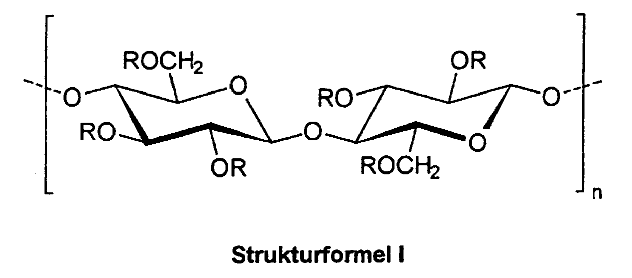 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das NatriumSalz des Glykolsäureethers der Cellulose, für welches R in Strukturformel I ein Wasserstoff und/oder CH₂-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×10⁶ bis 24×10⁶ produziert. Xanthan wird aus einer Kette mit β-1,4-gebundener Glucose (Cellulose) mit Seitenketten gebildet. Die Struktur der Untergruppen ("repeated units") besteht aus Glucose, Mannose, Glucuronsäure, Acetat und Pyruvat.

Erfindungsgemäß vorteilhafte Hydrokolloide sind ferner Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Carbopole sind Verbindungen der allgemeinen Strukturformel 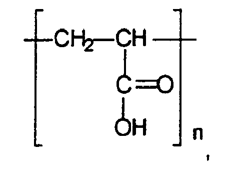 deren Molgewicht zwischen ca. 400 000 und mehr als 4 000 000 betragen kann. In die Gruppe der Carbopole gehören ferner Acrylat-Alkylacrylat-Copolymere, beispielsweise solche, die sich durch die folgende Struktur auszeichnen:

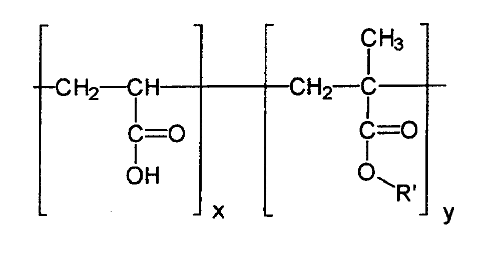

Darin stellen R' einen langkettigen Alkylrest und x und y Zahlen dar, welche den jeweiligen stöchiometrischen Anteil der jeweiligen Comonomere symbolisieren. Auch diese Carbopole sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984, wobei diese Verbindungen einzeln oder in beliebigen Kombinationen untereinander vorliegen können. Besonders bevorzugt sind Carbopol 981, 1382 und 5984 (sowohl einzeln als auch in Kombination mit weiteren Hydrokolloiden).

Ferner vorteilhaft im Sinne der vorliegenden Erfindung sind die den Acrylat-Alkylacrylat-Copolymeren vergleichbaren Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Die Gesamtmenge an einem oder mehreren Hydrokolloiden wird in den fertigen kosmetischen oder dermatologischen W/O-Pickering-Emulsionen vorteilhaft kleiner als 1,0 Gew.-%, bevorzugt zwischen 0,01 und 0,5 Gew.-%, bezogen auf das Gesamtgewicht der Zubereitungen gewählt.

Es ist ferner vorteilhaft, wenngleich nicht zwingend, wenn die erfindungsgemäßen Pikkering-Emulsionen weitere Hilfsstoffe enthalten, die die Stabilität dieser Zubereitungen zusätzlich erhöhen können, beispielsweise Stoffe, die gewählt werden aus der Gruppe der Wachse und/oder Ölverdickungsmittel sowie der Elektrolyte.

Es ist ferner vorteilhaft, wenn die erfindungsgemäßen Pickering-Emulsionen Hilfsstoffe enthalten, die zur Verminderung oder Verhinderung eines stumpfen oder trockenen Hautgefühls nach deren Auftragen beitragen können, wobei der hauptsächliche Zweck dieser Stoffe ein anderer sein kann. Vorzugsweise werden diese Stoffe beispielsweise gewählt aus der Gruppe der unsymmetrisch substituierten s-Triazinderivate, der Cyclodextrine, der Filmbildner und der polymeren Moisturizer, wobei diese Stoffe sowohl einzeln als auch im Gemisch vorliegen können.

Die kosmetischen Eigenschaften der erfindungsgemäßen Pickering-Emulsionen können zusätzlich beispielsweise noch dadurch verbessert werden, daß in der Ölphase auch Öle eingesetzt werden, welche eine Viskosität haben, die kleiner als 30 mPa·s, insbesondere kleiner als 20 mPa·s ist (bestimmt mit einem Rheometer der Firma Contraves (Rheomat 108E) bei einem Schergefälle von 500/s und einer Temperatur von 25 °C).

### Mikrofeine Partikel:

Der amphiphile Charakter der erfindungsgemäßen mikrofeinen Partikel zeigt sich beispielsweise darin, daß diese sowohl in Wasser als auch in Öl dispergierbar sind.

Es ist vorteilhaft, den mittleren Partikeldurchmesser der verwendeten Partikel zwischen 1 nm und 200 nm, besonders vorteilhaft zwischen 5 nm und 100 nm zu wählen.

Es ist ferner vorteilhaft, die Konzentration aller erfindungsgemäßen amphiphilen Partikel größer als 0,1 Gew.-%, besonders vorteilhaft zwischen 0,1 Gew.-% und 30 Gew.-%, bezogen auf das Gesamtgewicht der Zubereitungen, zu wählen.

Vorteilhaft im Sinne der vorliegenden Erfindung sind alle Partikel, die geeignet sind, Pikkering-W/O-Emulsionen zu stabilisieren. Im wesentlichen unerheblich für die vorliegende Erfindung ist es, in welcher der gegebenenfalls natürlich vorkommenden Modifikationen die Partikel vorliegen.

Vorzugsweise werden zur Stabilisierung der Pickering-Emulsionen unbehandelte, nahezu reine Pigmentpartikel verwendet, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Pickering-Emulsionen im Sinne der vorliegenden Erfindung werden ebenfalls vorteilhaft durch anorganische Pigmente stabilisiert, die oberflächlich wasserabweisend behandelt ("gecoatet") sind, wobei gleichzeitig der amphiphile Charakter gebildet werden bzw. erhalten bleiben soll.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid, als auch durch Kombination mehrerer Pigmenttypen innerhalb einer Zubereitung.

Vorzugsweise werden die erfindungsgemäßen Pickering-Emulsionen ferner durch Bornitridpartikel stabilisiert, beispielsweise durch die im folgenden aufgelisteten Bornitride:

| **Handelsname** | **Erhältlich bei** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN^{®} | Carborundum |
| Wacker-Bomitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Pickering-Emulsionen im Sinne der vorliegenden Erfindung werden ebenfalls vorteilhaft durch Bornitridpartikel stabilisiert, die oberflächlich wasserabweisend behandelt ("gecoatet") sind, wobei gleichzeitig der amphiphile Charakter gebildet werden bzw. erhalten bleiben soll.

Eine vorteilhafte Beschichtung der Bornitridpartikel besteht aus Dimethylpolysiloxan (Dimethicon). Vorteilhaft sind beispielsweise die bei der Firma Carborundum unter der Handelsbezeichnung Très BN^{®} UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN^{®} UHP 1107 erhältlichen.

Es ist ferner vorteilhaft, die erfindungsgemäßen Pickering-Emulsionen durch mikrofeine Polymerpartikel zu stabilisieren.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide, Polyacrylate und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol^{®} 1002 (Polyamid 6) und Orgasol^{®} 2002 (Polyamid 12) von der Firma ELF ATO-CHEM.

Weitere vorteilhafte Polymerpartikel sind mikrofeine Polymethacrylate. Solche Partikel sind beispielsweise unter der Handelsbezeichnung POLYTRAP^{®} bei der Firma DOW CHEMICAL erhältlich.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus TiO₂, ZrO₂ oder auch weiteren Polymeren, wie beispielsweise Polymethylmethacrylat.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 µm, besonders vorteilhaft kleiner als 50 µm zu wählen. Dabei ist es im wesentlichen unerheblich, in welcher Form (Plättchen, Stäbchen, Kügelchen etc.) die verwendeten Polymerpartikel vorliegen.

Desweiteren ist es vorteilhaft, die erfindungsgemäßen Pickering-Emulsionen durch modifizierte Polysaccharide zu stabilisieren.

Modifizierte Polysaccharide im Sinne der vorliegenden Erfindung sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Solche Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel 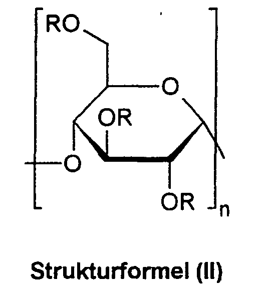 worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (II), worin R sich durch die folgende Struktur auszeichnet 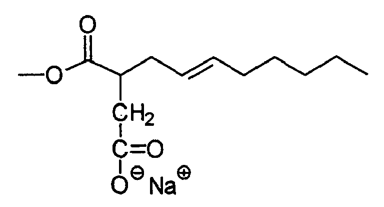 und welches z. B. unter der Handelsbezeichnung Amiogum^{®} 23 bei der Firma CERE-STAR erhältlich ist sowie Aluminium Stärke Octenylsuccinate, insbesondere die unter den Handelsbezeichnungen Dry Flo^{®} Elite LL und Dry Flo^{®} PC bei der Firma CERE-STAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen.

Die Liste der genannten modifizierten Polysaccharide, die erfindungsgemäße Pickering-Emulsionen stabilisieren können, soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Vorteilhaft zur Stabilisierung von W/O-Pickering-Emulsionen sind insbesondere auch Magnesiumsilicate (auch: Talkum), beispielsweise die unter der Handelsbezeichnung Talkum Micron bei der Firma Grolmann erhältlichen.

Der Wasserphasenanteil der erfindungsgemäßen W/O-Pickering-Emulsionen wird vorzugsweise aus dem Bereich von 0,5 bis 75 Gew. % gewählt, bezogen auf das Gesamtgewicht der Formulierungen.

Es ist ferner vorteilhaft, wenngleich nicht zwingend, die erfindungsgemäßen mikrofeinen Partikel mit weiteren amphiphilen Partikeln zu kombinieren, welche gegebenenfalls auch zur Stabilisierung der Pickering-Emulsionen beitragen können.

Solche Partikel sind beispielsweise Titandioxidpigmente, die mit Octylsilanol beschichtet sind, und/oder Siliciumdioxidpartikel, die oberflächlich wasserabweisend behandelt sind. Geeignete Siliciumdioxidpartikel sind beispielsweise sphärische Polyalkylsilsesquioxan-Partikel, wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich. Geeignete Titandioxidpartikel sind unter der Handelsbezeichnung T805 ebenfalls bei der Firma Degussa erhältlich.

### Ölphase

Die Ölphase der erfindungsgemäßen O/W-Emulsionen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase der erfindungsgemäßen O/W-Emulsionen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Ferner kann die Ölphase der erfindungsgemäßen O/W-Emulsionen ebenfalls vorteilhaft, wenngleich nicht zwingend auch unpolare Öle enthalten, beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Die erfindungsgemäßen Pickering-Emulsionen können als Grundlage für kosmetische oder dermatologische Formulierungen dienen. Diese können wie üblich zusammengesetzt sein und beispielsweise zur Behandlung und der Pflege der Haut, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, weitere Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können die erfindungsgemäßen Pickering-Emulsionen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B.

Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl-und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Günstig sind auch kosmetische und dermatologische Zubereitungen, die in der Form eines Sonnenschutzmittels vorliegen. Vorzugsweise enthalten diese mindestens eine UV-A-Filtersubstanz und/oder mindestens eine UV-B-Filtersubstanz und/oder mindestens ein weiteres anorganisches Pigment aus der Gruppe der Oxide des Eisens, Zirkoniums, Siliciums, Mangans, Aluminiums, Cers und Mischungen davon sowie Abwandlungen, bei denen die Oxide die aktiven Agentien sind.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure: 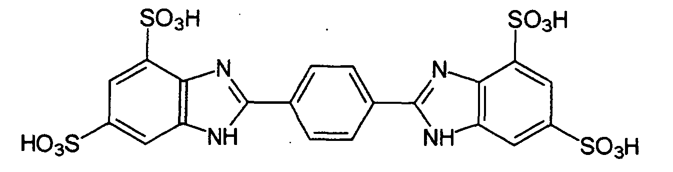 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz: 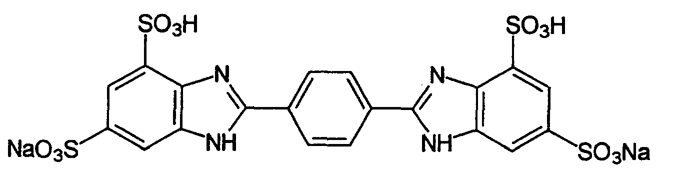 sowie das 1,4-di(2-oxo-10-Sulfo-3-bomylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

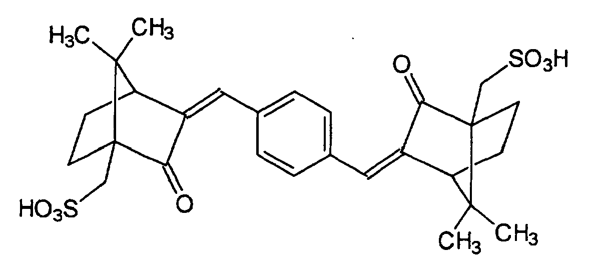

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter und/oder UV-B-Filtersubstanzen sind beispielsweise Bis-Resorcinyltriazinderivate mit der folgenden Struktur: 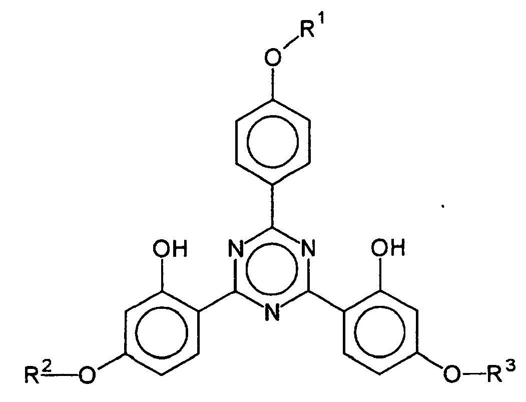 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin, welches von der BASF Aktiengesellschaft unter der Warenbezeichnung UVINUL® T 150 vertrieben wird.

Auch andere UV-Filtersubstanzen beschrieben, welche das Strukturmotiv 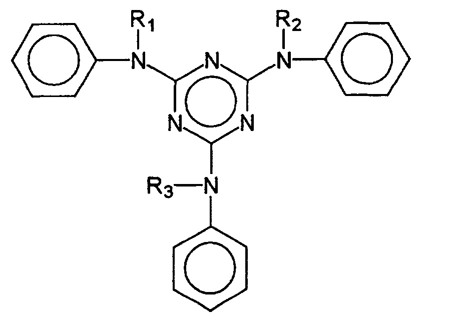 aufweisen sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 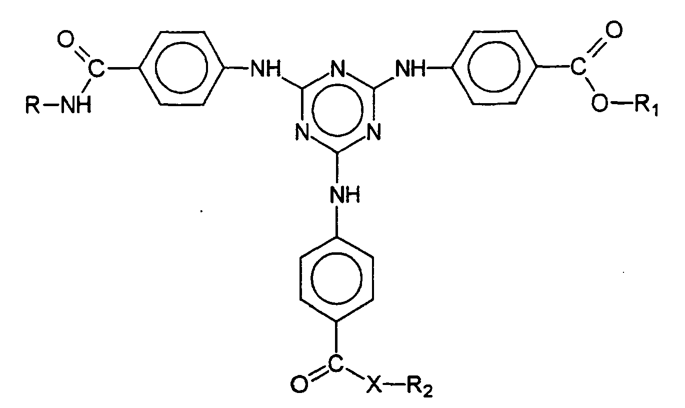 wiedergegeben wird, wobei
- R: einen verzweigten oder unverzweigten C₁-C₁₈Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 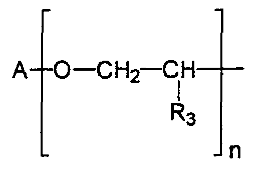 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 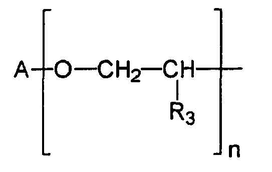 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
wenn X ein Sauerstoffatom darstellt.

Ein Beispiel für solche unsymmetrisch substituierte s-Triazine stellt das Dioctylbutylamidotriazon dar.

Auch andere UV-Filtersubstanzen, deren Einarbeitung in kosmetische oder dermatologische Lichtschutzformulierungen bisher Probleme aufwies, sind bekannt. So werden in der Europäischen Offenlegungsschrift 775 698 Bis-Resorcinyltriazinderivate beschrieben, deren chemische Struktur durch die generische Formel 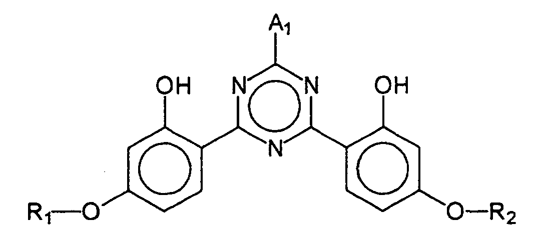 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(2-Ethyl-hexyloxy)-2-hydroxy]-phenyl}-6-(4-methoxyphenyl)-1,3,5-triazin, das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein weiterer vorteilhafter UV-Filter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 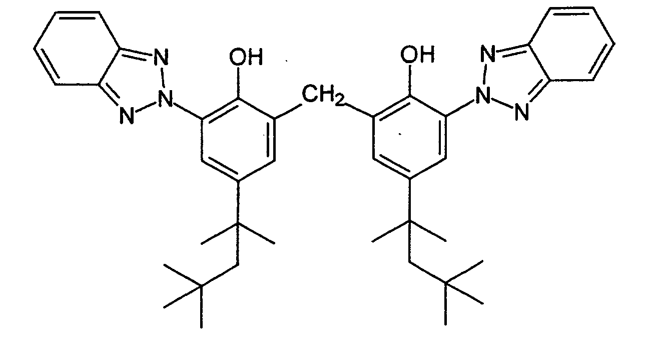 gekennzeichnet ist.

Vorteilhaft im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 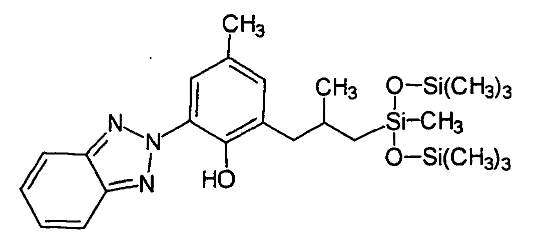 gekennzeichnet ist.

Die UV-B-Filter können öllöslich oder wasserlöslich sein. Vorteilhafte öllösliche UV-B-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist und sich durch folgende Struktur auszeichnet: 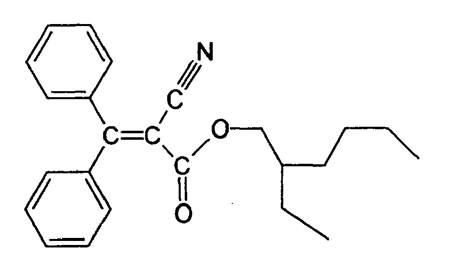 Es kann auch von erheblichem Vorteil sein, polymergebundene oder polymere UV-Filter substanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hektorit, Bentonit, Smectit, ferner beispielsweise Zinksalze der Ricinolsäure. Keimhemmende Mittel sind ebenfalls geeignet, in die erfindungsgemäßen W/O-Emulsionsstifte eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quatemäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Liste der genannten Wirkstoffe bzw. Wirkstoffkombinationen soll selbstverständlich nicht limitierend sein.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

| | **1** | **2** | **3** |
|---|---|---|---|
| | **W/O** | **W/O** | **W/O** |
| Titandioxid (Eusolex T2000) | 2 | 4 | 6 |
| Zinkoxid | 5 | | 4 |
| Titandioxid (Titandioxid T805) | | | 2 |
| Silica (Aerosil R972) | | 1 | 0,5 |
| Talkum (Talkum Micron) | | 0,5 | |
| Bornitrid | | 2 | |
| Hydroxystearyl Hydroxystearat (Elfacos C26) | 2 | | 2 |
| C₂₀₋₄₀ Alkylstearat (Kesterwachs K82) | 1 | 1 | |
| C₁₆₋₃₈ Alkylhydroxystearoylstearat (Kesterwachs K80P) | | 2 | |
| Behenoxy Dimethicon (Abil Wax 2440) | | | 5 |
| Polyisobuten (Rewopal PIB 1000) | 5 | | |
| Caprylic/Capric Triglycerid | 5 | 5 | 5 |
| Octyldodecanol | 5 | | 5 |
| Mineralöl | 10 | | |
| Butylen Glycol Caprylat/Caprat | | 10 | 10 |
| C₁₂₋₁₅ Alkyl Benzoat | 10 | 10 | 10 |
| Dimethicon | | 2 | 3 |
| Hydriertes Polyisobuten (Polysynlan) | 2 | | |
| Methylbenzyliden Campher | | 3 | |
| Octyltriazon | | 1 | |
| Dibenzoylmethan | | 2 | |
| Dioctyl Butamido Triazon (UVASORB HEB) | | 2 | |
| Konservierung | 0,5 | 0,5 | 0,5 |
| Glycerin | 5 | 10 | 3 |
| Biosaccharid Gel (Fucogel 1000) | | | 5 |
| NaCl | 1 | | 1 |
| MgSO₄ | | 0,5 | |
| Phenylbenzimiazol Sulfonsäure | | 1 | |
| Carbomer (Carbopol 981) | | 0,1 | |
| C₁₀₋₃₀ Alkyl Acrylat Crosspolymer (Pemulen TR1) | | | 0,01 |
| Xanthan Gummi | 0,05 | | |
| Natriumcarboxymethylcellulose (Natrosol Plus 330 CS) | | 0,05 | 0,01 |
| NaOH 45%ige Lösung in Wasser | | 0,3 | 0,01 |
| EDTA-Lösung | | 1 | |
| Wasser | ad 100 | ad 100 | ad 100 |

## Patentansprüche

1. Pickering-Emulsionen, welche feindisperse Systeme vom Typ Wasser-in-Öl darstellen, enthaltend
(1) eine Ölphase,
(2) eine Wasserphase,
(3) mindestens einen Typ mikrofeiner Partikel, die
a) eine mittlere Partikelgröße von weniger als 200 nm haben, die
b) sowohl hydrophile als auch lipophile Eigenschaften zeigen, welche also amphiphilen Charakter besitzen und sowohl in Wasser als auch in Öl dispergierbar sind und die
c) gegebenenfalls oberflächlich beschichtet sind,
(4) mindestens ein Hydrokolloid und
(5) höchstens 0,5 Gew.-% eines oder mehrerer Emulgatoren.

2. Pickering-Emulsionen nach Anspruch 1, **dadurch gekennzeichnet, dass** sie emulgatorfrei sind.

3. Pickering-Emulsionen nach einem der vorhergehenden Ansprüche, **dadurch gekennzeichnet, dass** der Gehalt der verwendeten Partikel zwischen 0,1 Gew.-% und 30 Gew.-% ist, bezogen auf das Gesamtgewicht der Zubereitungen.

4. Pickering-Emulsionen nach einem der vorhergehenden Ansprüche, **dadurch gekennzeichnet, dass** der Partikeldurchmesser der verwendeten Partikel zwischen 5 nm und 100 nm liegt.

5. Pickering-Emulsionen nach einem der vorhergehenden Ansprüche, **dadurch gekennzeichnet, dass** die verwendeten Partikel gewählt werden aus der Gruppe bestehend aus amphiphilen Metalloxiden, Bornitrid, mikrofeinen Polymerpartikeln und modifizierten Polysacchariden, wobei die Partikel sowohl einzeln als auch im Gemisch vorliegen können.

6. Pickering-Emulsionen nach einem der vorhergehenden Ansprüche, **dadurch gekennzeichnet, dass** die verwendeten Partikel oberflächlich wasserabweisend behandelt sind, wobei der amphiphile Charakter der Partikel gebildet wird bzw. erhalten bleibt.

7. W/O-Pickering-Emulsionen nach einem der vorhergehenden Ansprüche, **dadurch gekennzeichnet, dass** die Gesamtmenge an einem oder mehreren Hydrokolloiden in den fertigen kosmetischen oder dermatologischen Zubereitungen zwischen 0,01 und 0,5 Gew.-% gewählt wird, bezogen auf das Gesamtgewicht der Zubereitungen.

8. Pickering-Emulsionen nach einem der vorhergehenden Ansprüche, **dadurch gekennzeichnet, dass** das oder die Hydrokolloide gewählt werden aus der Gruppe der Cellulosederivate, insbesondere aus der Gruppe, die gebildet wird aus (Hydroxypropyl)methylcellulosen, Natriumcarboxymethylcellulose und Xanthan Gummi.

9. Pickering-Emulsionen nach einem der vorhergehenden Ansprüche, **dadurch gekennzeichnet, dass** das oder die Hydrokolloide gewählt werden aus der Gruppe der Acrylat-Alkylacrylat-Copolymere und der Copolymere aus C₁₀₋₃₀-Alkylacrylaten und einem oder mehreren Monomeren der Acrylsäure, der Methacrylsäure oder deren Ester.

10. Pickering-Emulsionen nach einem der vorhergehenden Ansprüche, **dadurch gekennzeichnet, dass** sie Hilfsstoffe enthalten, die zur Verminderung oder Verhinderung eines stumpfen oder trockenen Hautgefühls nach deren Auftragen beitragen können.

## Claims

1. Pickering emulsions, which are finely disperse systems of the water-in-oil type, comprising
(1) an oil phase,
(2) a water phase,
(3) at least one type of microfine particles which
a) have an average particle size of less than 200 nm, which
b)exhibit both hydrophilic and also lipophilic properties, which thus have amphiphilic character and are dispersible both in water and also in oil and which
c) are optionally surface-coated,
(4) at least one hydrocolloid and
(5) at most 0.5% by weight of one or more emulsifiers.

2. Pickering emulsions according to Claim 1, **characterized in that** they are emulsifier-free.

3. Pickering emulsions according to one of the preceding claims, **characterized in that** the content of the particles used is between 0.1% by weight and 30% by weight, based on the total weight of the preparations.

4. Pickering emulsions according to one of the preceding claims, **characterized in that** the particle diameter of the particles used is between 5 nm and 100 nm.

5. Pickering emulsions according to one of the preceding claims, **characterized in that** the particles used are chosen from the group consisting of amphiphilic metal oxides, boron nitride, microfine polymer particles and modified polysaccharides, where the particles may be present either individually or in a mixture.

6. Pickering emulsions according to one of the preceding claims, **characterized in that** the particles used are surface-coated to repel water, with the amphiphilic character of the particles being formed and/or retained.

7. W/O Pickering emulsions according to one of the preceding claims, **characterized in that** the total amount of one or more hydrocolloids in the finished cosmetic or dermatological preparations is chosen between 0.01 and 0.5% by weight, based on the total weight of the preparations.

8. Pickering emulsions according to one of the preceding claims, **characterized in that** the hydrocolloid or hydrocolloids are chosen from the group of cellulose derivatives, in particular from the group which is formed by (hydroxypropyl)methylcelluloses, sodium carboxymethylcellulose and xanthan gum.

9. Pickering emulsions according to one of the preceding claims, **characterized in that** the hydrocolloid or hydrocolloids are chosen from the group of acrylate-alkyl acrylate copolymers and copolymers of C₁₀₋₃₀-alkyl acrylates and one or more monomers of acrylic acid, methacrylic acid or esters thereof.

10. Pickering emulsions according to one of the preceding claims, **characterized in that** they comprise auxiliaries which can contribute to reducing or preventing a rough or dry skin feel after they have been applied.

## Revendications

1. Emulsions de Pickering, qui représentent des systèmes finement dispersés du type eau-dans-huile ou huile-dans-eau, contenant
(1) une phase huileuse,
(2) une phase aqueuse,
(3) au moins un type de particules microfines qui
a) ont une taille moyenne de particule inférieure à 200 nm, qui
b) présentent aussi bien des propriétés hydrophiles que des propriétés lipophiles, qui ont donc un caractère amphiphile et sont dispersables aussi bien dans l'eau que dans l'huile et qui
c) sont éventuellement enrobées en surface,
(4) au moins un hydrocolloïde et
(5) au maximum 0,5 % en poids d'un ou plusieurs émulsifiants.

2. Emulsions de Pickering selon la revendication 1, **caractérisées en ce qu'**elles sont exemptes d'émulsifiant.

3. Emulsions de Pickering selon l'une quelconque des revendications précédentes, **caractérisées en ce que** la teneur en les particules utilisées est comprise entre 0,1 % en poids et 30 % en poids, par rapport au poids total des préparations.

4. Emulsions de Pickering selon l'une quelconque des revendications précédentes, **caractérisées en ce que** le diamètre de particule des particules utilisées est compris entre 5 nm et 100 nm.

5. Emulsions de Pickering selon l'une quelconque des revendications précédentes, **caractérisées en ce que** les particules utilisées sont choisies dans le groupe constitué par des oxydes métalliques amphiphiles, le nitrure de bore, des particules de polymère microfines et des polysaccharides modifiés, les particules pouvant être présentes aussi bien individuellement qu'en mélange.

6. Emulsions de Pickering selon l'une quelconque des revendications précédentes, **caractérisées en ce que** les particules utilisées présentent en surface un traitement hydrofuge, le caractère amphiphile des particules étant acquis ou conservé.

7. Emulsions de Pickering E/H selon l'une quelconque des revendications précédentes, **caractérisées en ce que** la quantité totale d'un ou de plusieurs hydrocolloïdes dans les préparations cosmétiques ou dermatologiques finies est choisie comprise entre 0,01 et 0,5 % en poids, par rapport au poids total des préparations.

8. Emulsions de Pickering selon l'une quelconque des revendications précédentes, **caractérisées en ce que** le ou les hydrocolloïdes sont choisis parmi le groupe des dérivés de la cellulose, en particulier parmi le groupe qui est constitué d'(hydroxypropyl)méthylcelluloses, de la carboxyméthylcellulose sodique et de la gomme de xanthane.

9. Emulsions de Pickering selon l'une quelconque des revendications précédentes, **caractérisées en ce que** le ou les hydrocolloïdes sont choisis parmi le groupe des copolymères acrylate/acrylate d'alkyle et des copolymères d'acrylates d' alkyle en C₁₀₋₃₀ et d' un ou de plusieurs monomères de l'acide acrylique, de l'acide méthacrylique et de leurs esters.

10. Emulsions de Pickering selon l'une quelconque des revendications précédentes, **caractérisées en ce qu'**elles contiennent des adjuvants qui peuvent contribuer à diminuer ou empêcher une sensation sèche ou pâteuse sur la peau après leur application.
